# EUROPEAN PATENT APPLICATION

(11) **EP 4 644 543 A1**
(43) Date of publication of application: **05.11.2025**
(21) Application number: 22969812.1
(22) Date of filing: 30.12.2022
(51) Int. Cl.: C12N 15/00, C12Q 1/6837, C12Q 1/6874

(54) **SPLINT SEQUENCE AND USE THEREOF IN NUCLEIC ACID CAPTURE**

(71) Applicant: BGI Shenzhen, Shenzhen, Guangdong 518083 (CN); BGI Shenzhen Co., Ltd, Shenzhen, Guangdong 518083 (CN)
(72) Inventor: HE, Ying, Shenzhen, Guangdong 518083 (CN); ZHAO, Yu, Shenzhen, Guangdong 518083 (CN); LIAO, Sha, Shenzhen, Guangdong 518083 (CN); CHEN, Ao, Shenzhen, Guangdong 518083 (CN); ZHANG, Wenwei, Shenzhen, Guangdong 518083 (CN)
(74) Representative: Tomkins & Co
(86) International application number: PCT/CN2022/144098
(87) International publication number: WO 2024/138670

(57) **Abstract**

Disclose in the present invention are a splint sequence and a use thereof in nucleic acid capture. The first base at the 5' end of the splint sequence is U. During nucleic acid capture, by using the splint sequence provided by the present invention, the effect of effectively reducing the number of adapters generated by the splint sequence in a sequencing library can be achieved, the mapping rate of sequencing data can be improved, and the sequencing costs can be reduced.

## Description

### TECHNICAL FIELD

The present disclosure belongs to the field of genetics, and specifically relates to a splint sequence and a use thereof in nucleic acid capture.

### BACKGROUND

Based on spatial transcriptomics of random probe capture of RNA, random probes (e.g., 6N) are generally used in the prior art to capture RNA in sections, followed by reverse transcription to obtain whole transcriptome information.

The process in the prior art generally involves subjecting a tissue section attached to the chip surface to fixation and permeabilization, followed by the addition of random probes comprising fixed nucleic acid sequences at the 5' end for hybridization, and then performing a reverse transcription (hereafter referred to as RT) reaction. After the reaction is completed, a ligation reaction is performed. The gap generated between the 3' end of the oligonucleotide sequence fixed on the chip and the 5' end of the random probe can be filled by T4 ligase, followed by high-throughput sequencing to obtain spatial location information for RNA in the section. However, the method of RT followed by T4 ligation results in the loss of captured cDNA information. Additionally, separating RT and T4 ligation into two steps requires RT to be performed at 42°C for at least 3 hours and T4 ligation to be performed at 16°C overnight, necessitating the use of two types of constant temperature equipment and being time-consuming.

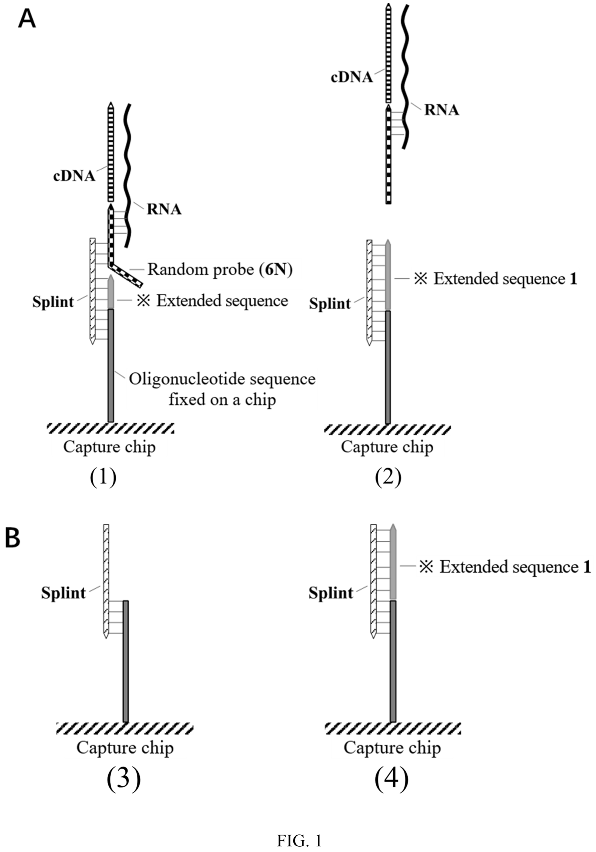

As shown in FIG. 1A and FIG. 1B, either the case where the T4 ligase fails to timely fill the nick gap or the case where there might be a splint that fails to hybridize with the random probe (including 6N) first leads to alterations in the probe sequence fixed on the chip. The fixed oligonucleotide sequence on the random probe is not able to be hybridized to the splint sequence with the extended capture probe, thereby preventing ligation and causing the original function of the extended oligonucleotide strand to be lost.

As shown in FIG. 1C, the method is a common method for adding sequencing adapters in high-throughput sequencing library preparation, by which a known fixed oligonucleotide sequence is added to the 3' end of cDNA, and the library is amplified using primers. Finally, the oligonucleotide strand fixed on the capture chip undergoes changes in the presence of RTase and sequencing adapters are added, resulting in a final sequencing library comprising a large number of nucleotide strands containing only the splint sequence.

On one hand, the aforementioned method results in the inability of the oligonucleotide strand on the capture chip to ligate the captured cDNA, rendering it a useless nucleic acid strand, which leads to the reduction of cDNA that can be eventually fixed on the chip, the loss of numerous transcripts from genes, and the inability to obtain the true RNA expression profile of the section. On the other hand, the sequencing data that can be mapped to the genome is limited (less than 10% of the valid sequencing data), and the issue is particularly pronounced when using the direct circularization-based library preparation method. Taking the MGISEQ-2000RS sequencer as an example, the library obtained by the aforementioned method generates approximately 220M valid sequencing data per lane, of which only approximately 10M can be mapped to the genome, greatly increasing the sequencing cost of the technical approach.

### SUMMARY

To address the shortcomings of the prior art that a large number of splint-induced adapters are generated in nucleic acid capture, the present disclosure provides a splint sequence and a use thereof in nucleic acid capture. Specifically, the 5' end of the splint sequence is uracil U (5'-/rU/SEQ ID NO: 5-3'). The oligonucleotide sequence fixed on the chip, after extension using the splint sequence as a template, fails to hybridize with the TSO at the 5' end of the splint sequence, thereby avoiding amplification and sequencing.

The first base at the 5' end of the splint sequence (5'-SEQ ID NO: 3-3') is G, and the terminal transferase activity of RTase tends to further polymerize base C at the 3' end of extended sequence 1 (FIG. 1C); whereas 5'-/rU/SEQ ID NO: 5-3' can prevent the sequence fixed on the chip from incorporating the sequencing adapter by extension, thereby ensuring that the sequence is not present in the sequencing library.

To address the shortcomings of the prior art, the first aspect of the present disclosure provides a splint sequence, wherein the first base at the 5' end of the splint sequence is U; preferably, the nucleotide sequence following the first base at the 5' end of the splint sequence is as shown in SEQ ID NO: 5.

The second aspect of the present disclosure provides a nucleic acid capture kit, comprising the splint sequence as described in the first aspect of the present disclosure. The nucleic acid capture kit further comprises at least one of the reagents used in conventional nucleic acid capture kits in the art, such as a random probe, a T4 ligase, a reverse transcriptase, a buffer for ligation, or a buffer for reverse transcription.

The third aspect of the present disclosure provides a use of the splint sequence as described in the first aspect of the present disclosure in nucleic acid capture.

The fourth aspect of the present disclosure provides a method for capturing nucleic acid, comprising reverse transcribing an RNA in a sample captured by a random probe into a cDNA, and ligating the cDNA to the random probe by means of hybridization of the splint sequence as described in the first aspect of the present disclosure to the random probe and a fixed oligonucleotide sequence, to form a long single-stranded nucleotide sequence containing the oligonucleotide sequence, the random probe, and the cDNA in the 5' to 3' direction, wherein the reverse transcription and the ligation are performed in the same reaction system.

In some embodiments, the method comprises the following steps:
(1) hybridizing the random probe to the splint sequence; wherein the 5' end of the random probe is partially complementary to the 5' end of the splint sequence;
(2) hybridizing the random probe to the RNA in the sample; wherein the 3' end of the random probe is partially complementary to the 3' end of the RNA;
(3) hybridizing the splint sequence to the fixed oligonucleotide sequence;
   wherein steps (1), (2), and (3) have no temporal sequence and can be performed simultaneously in the same reaction system;
   preferably, the oligonucleotide sequence is fixed on a capture chip;
(4) in the same reaction system, synthesizing the cDNA using the RNA in the complex formed by simultaneous hybridization in steps (1), (2), and (3) as a template, and ligating the fixed oligonucleotide sequence to the random probe in the complex, allowing the fixed oligonucleotide sequence, the random probe, and the cDNA to form a long single-stranded nucleotide sequence containing cDNA in the 5' to 3' direction.

In some embodiments, the capture chip is reacted at 30 to 45°C, such as 37°C, for 3 to 24 hours, such as 3 to 5 hours.

Preferably, in step (1), the random probe comprises 6 to 20 N.

In some embodiments, the method further comprises:
(i) mixing a random probe and the splint sequence with 5× SSC, performing a hybridization in an incubator at 50 to 60°C, such as 55°C, for 5 to 30 minutes, such as 10 minutes, to form a primer hybridization mixture, removing and cooling the primer hybridization mixture, and adding 2 to 15% by volume, such as 5% by volume of RNase inhibitor; or
(i') mixing the random probe and the splint sequence in a solution, gradient cooling to room temperature, followed by cooling to 0 to 4°C to obtain a primer hybridization mixture, and mixing the primer hybridization mixture with 5× SSC and 2 to 15% by volume, such as 5% by volume of RNase inhibitor.

In some embodiments, after step (i) or step (i'), the method further comprises:
(ii) aspirating the liquid from the surface of a capture chip, washing the capture chip with a mixture 2, adding the primer hybridization mixture to the chip surface, performing a hybridization at room temperature for 15 to 60 minutes, and washing the capture chip again with a mixture 2; the mixture 2 contains 5× SSC and RNase inhibitor.

In some embodiments, in step (i'), the gradient cooling is performed from 90 to 100°C to 20 to 28°C at a rate of 1 to 5°C per minute;
in step (i) or (i'), the random probe is 6N;
in step (i) or (i'), the molar ratio of the random probe to the splint sequence is (1 to 10):(1 to 10); and/or,
in step (ii), the volume ratio of 5× SSC to RNase inhibitor in the mixture 2 is (5 to 30):1.

In some embodiments, in step (i'), the gradient cooling is performed from 95°C to 25°C at a rate of 2°C per minute;
in step (i) or (i'), the molar ratio of the random probe to the splint sequence is 1:1; and/or,
in step (ii), the volume ratio of 5× SSC to RNase inhibitor in the mixture 2 is 19:1.

In some embodiments, the capture chip comprises a capture probe, wherein the capture probe comprises spatial barcode location information and a fixed oligonucleotide sequence; the fixed oligonucleotide sequence is hybridized to the 3' end of the splint sequence. Furthermore, the fixed oligonucleotide sequence may be ligated to the 5' end of the random probe.

In some embodiments, the spatial barcode location information is Nm, the fixed oligonucleotide sequence is as shown in SEQ ID NO: 1, the capture probe is 5'Nm-SEQ ID NO: 1-3', and the m is 8 to 30.

In some embodiments, the m is 25 or more.

The capture chip further comprises a fixed oligonucleotide sequence' for PCR amplification and Read 1 sequencing of a cDNA library.

In some embodiments, the method further comprises the following steps:
(a) after completing step (ii), aspirating the liquid from the surface of the capture chip, adding a mixture 1 containing a reverse transcriptase and a T4 ligase, and reacting the capture chip in an incubator for 3 to 24 hours;
(b) removing the tissue sample from the capture chip; and/or,
(c) recovering cDNA from the capture chip; optionally further comprising the steps of cDNA amplification and purification.

Preferably, the mixture 1 contains a reverse transcription reagent, 3 to 25 mM of MgCl₂, 1 to 50 mM of DTT, and 1 mM or more of ATP;
the volume ratio of the reverse transcriptase to the T4 ligase is 2:1 or 1:1; and/or,
the incubator has a temperature of 35 to 42°C.

The fifth aspect of the present disclosure provides a high-throughput sequencing method, comprising the following steps:
(I) obtaining a long single-stranded nucleotide sequence containing cDNA to be sequenced according to the method as described in the third aspect of the present disclosure;
(II) constructing a cDNA library;
(III) sequencing the library and analyzing the obtained data.

In some embodiments, step (I) is followed by the steps of cDNA fragmentation, amplification, and purification; and/or, in step (II), the cDNA library is circularized to obtain a circularized library of DNB.

On the basis of common sense in the art, the above preferred conditions can be combined arbitrarily to obtain various preferred examples of the present disclosure.

The reagents and raw materials used in the present disclosure are commercially available.

The positive and progressive effects of the present disclosure are as follows:

By using the splint sequence (5'-/rU/SEQ ID NO: 5-3') provided by the present disclosure, the effect of effectively reducing the number of adapters generated by the splint sequence in a sequencing library can be achieved, the mapping rate of sequencing data can be improved, and the sequencing costs can be reduced.

### BRIEF DESCRIPTION OF THE DRAWINGS

FIG. 1 shows the principle of splint-induced adapters (the arrowhead indicates the 3' end of the nucleic acid strand).

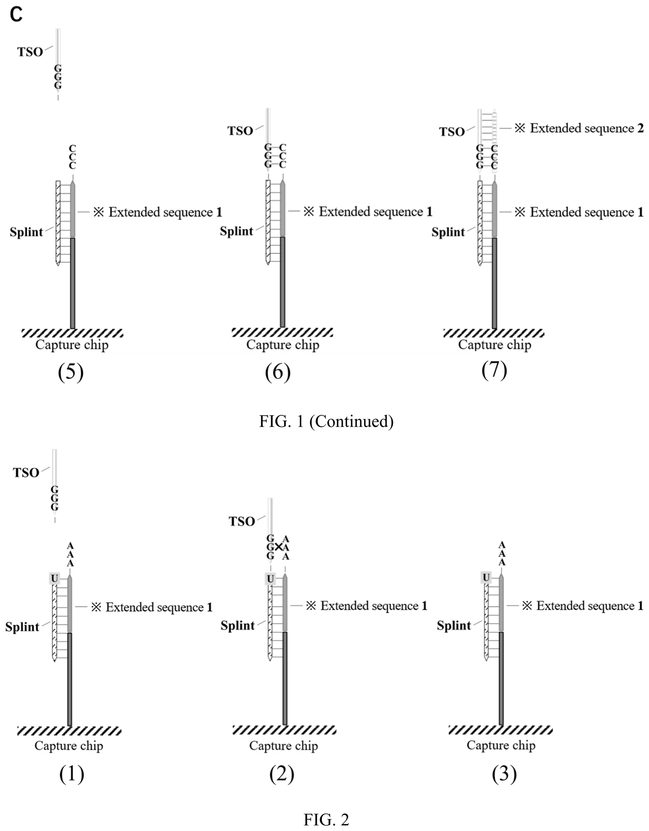

FIG. 2 shows the principle of reducing splint-induced adapters (the arrowhead indicates the 3' end of the nucleic acid strand).
FIG. 3 shows a comparison of the adapter data between the splint (5'-SEQ ID NO: 3-3') and the splint (5'-/rU/SEQ ID NO: 5-3').

### DETAILED DESCRIPTION OF THE PREFERRED EMBODIMENT

### Example 1

### 1.1 Sectioning of samples

(1) Sectioning of frozen OCT-embedded samples: The cryostat chamber (-20°C) and the specimen head (-10°C to -15°C, adjusted according to the actual operation process) were pre-cooled, and experimental tools such as brushes and blades were pre-cooled in the chamber at -20°C in advance. Frozen OCT-embedded tissue blocks were removed from a -80°C freezer and equilibrated in the cryostat, then fixed onto the specimen holder using liquid-state OCT at room temperature, and frozen until the OCT solidified. The tissue block was trimmed with a pre-cooled blade as needed, followed by sectioning.
(2) Sectioning of paraffin-embedded samples: A Leica paraffin microtome was used, with the thickness of paraffin sections set to 5 µm. The paraffin-embedded blocks were appropriately trimmed before sectioning.

### 1.2 RNA quality control

(1) Frozen OCT-embedded samples: 10 to 20 tissue sections of 10 µm thickness were cut and placed into a 1.5 mL EP tube pre-chilled to -20°C. Total RNA was extracted using the RNeasy Mini Kit (QIAGEN) and RNA quality was assessed using the Agilent RNA 6000 Pico Kit (Agilent). Samples with an RNA integrity number (RIN) of 7 or more were eligible for subsequent experiments.
(2) Paraffin-embedded samples: 2 to 4 sections of 5 µm thickness were cut and placed into a 1.5 mL RNase-free centrifuge tube. RNA was extracted using the RNeasy FFPE Kit and RNA quality was assessed using the Agilent RNA 6000 Pico Kit. Samples with a DV200 of 50% or more were eligible for subsequent experiments.

### 1.3 Chip processing and tissue mounting

(1) Frozen OCT-embedded samples: The capture chip was carefully gripped with forceps and placed into a new 24-well plate, and the number of the capture chip was recorded. The capture chip contained a capture probe comprising spatial barcode information and a fixed oligonucleotide sequence: 5'-N₂₅-TTGTCTTCCTAAGACCGCTTGG-3' (the underlined portion corresponds to SEQ ID NO: 1, and N₂₅ represents 25 consecutive Ns). The chip was washed twice with 400 µL of 0.1× SSC, followed by thorough washing with nuclease-free water (NF-H₂O), and dried at 37°C. A frozen OCT-embedded tissue section of 10 µm thickness was cut and flattened. The capture chip was gripped with forceps and swiftly brought into contact with the frozen section, allowing the tissue section to automatically adhere to the chip. The tissue-mounted chip was then baked at 37°C for 3 to 5 minutes.
(2) Paraffin-embedded samples: The chip was processed as described above. Paraffin-embedded samples were cut into sections of 5 to 8 µm and flattened in a water bath at 42°C. The section was mounted with a capture chip and baked at 60°C. The tissue-mounted chip was then immersed in a deparaffinization solution for 30 minutes, and the step was repeated once. After removal, the chip was blotted on absorbent paper, followed by immersion in 100% anhydrous ethanol for 5 minutes, and the step was repeated once. After removal, the chip was immersed in 96% ethanol for 5 minutes, and the step was repeated once.

### 1.4 Fixation (de-crosslinking) and permeabilization of tissue sections

(1) Frozen OCT-embedded samples: The chip was air-dried and fixed in a methanol solution pre-chilled to -20°C for 30 minutes. After removal, methanol was air-dried, and permeabilization reagent (prepared by mixing permease with 10 µL of 0.1 N HCl and 90 µL of NF-H₂O) was added. The chip was reacted in an incubator at 37°C for 10 to 13 minutes.
(2) Paraffin-embedded samples: The chip was air-dried and placed in a 24-well cell plate, and de-crosslinking buffer (prepared by mixing 380 µL of TE buffer at pH 9.0 with 20 µL of RNase inhibitor (RI)) was added. The chip was reacted in an incubator at 70°C for 1 hour. After the reaction was completed, the chip was removed, allowed to return to room temperature, and fixed in a methanol solution pre-chilled to -20°C for 20 minutes. After removal, methanol was air-dried, and permeabilization reagent (same as above) was added. The chip was reacted in an incubator at 37°C for 20 minutes.

### 1.5 Hybridization of random probe (6N) with splint

A primer hybridization mixture was prepared during the fixation of tissue sections. 5 µM random probe 6N (5'pho-SEQ ID NO: 4-3') and 5 µM splint (5'-/rU/SEQ ID NO: 5-3') were added to 5× SSC, mixed well, and hybridized in an incubator at 55°C for 10 minutes. After removal, the mixture was placed on ice, added with 5% by volume of RNase inhibitor (RI), mixed well, and placed on ice.

**Table 1. Random probe 6N and splint sequences and hybridization methods used in the present disclosure**

| | | Sequence | SEQ ID NO: |
|---|---|---|---|
| Comparative Examples 1 and 2 | Random probe 6N: | 5'pho- CCTCCGACTGTGTGACTTAGACTT GCACTTGATGTGCTNNNNNN-3' | 5'pho-SEQ ID NO: 2-3' |
| | splint: | 5'- CAAGTGCAAGTCTAAGTCACACA GTCGGAGGCCAAGCGGTCTTAG-3' | 5'-SEQ ID NO: 3-3' |
| Examples 1 and 2 | Random probe 6N: | 5'pho- TCTCCGACTGTGTGACTATGACTT GAAATTAATGTGCTNNNNNN-3' | 5'pho-SEQ ID NO: 4-3' |
| | splint: | 5'- /rU/AATTTCAAGTCATAGTCACACA GTCGGAGACCAAGCGGTCTTAG-3' | 5'-/rU/SEQ ID NO: 5-3' |
| Brief description of hybridization methods in Comparative Example 1 and Example 1 | | The random probe 6N and the splint were mixed at a molar ratio of 1:1 and hybridized at 55°C for 10 minutes. | |
| Brief description of hybridization methods in Comparative Example 2 and Example 2 | | The random probe 6N and the splint were mixed at a molar ratio of 1:1 and cooled from 95°C to 25°C at a rate of 2°C per minute. | |

| | | | |
|---|---|---|---|
| (pho- represents a phosphorylation modification; rU indicates that U is a ribonucleotide) | | | |

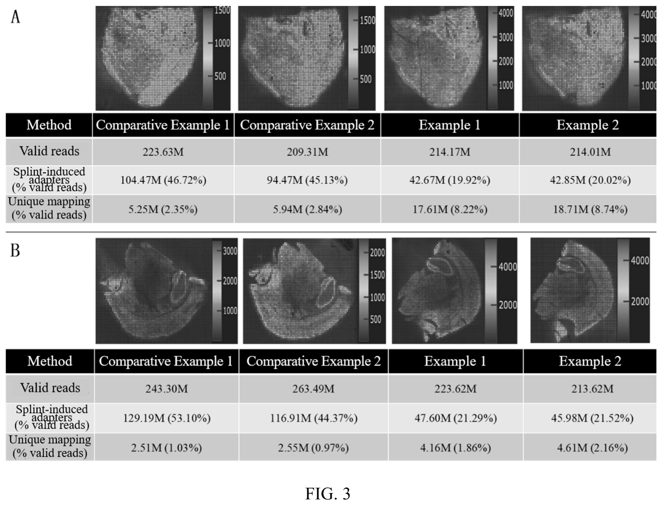

After the permeabilization reaction was completed, the chip was removed, and the liquid was aspirated from the chip surface. The chip was washed once with a mixture solution of 190 µL 5× SSC and 10 µL RI, added with the prepared primer hybridization mixture, and hybridized at room temperature for 15 minutes. The chip was then washed once with 5× SSC and RI. A primer hybridization mixture was obtained, as shown in Group 3 of FIG. 3.

### 1.6 Reverse transcription (RT) and T4 ligation

The liquid was aspirated from the chip surface, and a mixture of RTase and T4 ligase (containing RT reagent, 3 mM of MgCl₂, 10 mM of DTT, 1 mM of ATP, pH 8.3 at 25°C; 2.5 µL/100 µL of RTase and 5 µL/100 µL of T4 ligase) was added. The chip was reacted in an incubator at 37°C for 5 hours.

### 1.7 Tissue removal

After the reaction was completed, the chip was removed, and the liquid was aspirated from the chip surface. The chip was then washed once with NF-H₂O. Tissue removal solution was added, and the chip was reacted in an incubator at 55°C for 20 minutes. After removal of the chip, the chip surface was pipetted up and down, followed by pipetting up and down twice with NF-H₂O to completely remove the tissue.

### 1.8 cDNA recovery, amplification, and purification

(1) cDNA recovery: 400 µL/well of cDNA release solution was added to the reaction wells of the chip, which were sealed with sealing film. The chip was covered with a lid, and the edges were secured to prevent volatilization. The chip was reacted in an incubator at 55°C for 3 hours to release cDNA. The liquid from the reaction wells was transferred to a new 1.5 mL centrifuge tube. The chip was then rinsed with 350 µL/well of NF-H₂O, and the rinse solution was collected into the same centrifuge tube. VAHTS^{™} DNA Clean Beads (VAZYME) were mixed with the recovery solution at a ratio of 0.8:1 in the 1.5 mL centrifuge tube. The mixture was shaken and mixed well, and incubated at room temperature for 10 minutes. After a brief centrifugation, the EP tube was placed on a magnetic rack for 3 minutes until the solution was clarified, and the supernatant was discarded. 1 mL of freshly prepared 80% ethanol was added. The mixture was shaken and mixed well, followed by a brief centrifugation, and allowed to stand for 30 seconds. The supernatant was discarded, and the tube was washed again with 80% ethanol. The supernatant was discarded, and the mixture was air-dried at room temperature until the surface of the magnetic beads was free of reflection and showed no cracks. 42 µL of NF-H₂O was added for re-dissolution. The mixture was shaken and mixed well, and allowed to stand at room temperature for 5 minutes. After a brief centrifugation, the tube was placed on a magnetic rack for 3 to 5 minutes until the solution was clarified, and the supernatant was transferred to a new PCR tube.
(2) cDNA amplification: 58 µL of PCR mixture (containing 50 µL of cDNA HIFI Master Mix and 8 µL of cDNA Primers) was added to the PCR tube, totaling 100 µL, followed by PCR reaction. The steps of PCR reaction were as follows: i) 95°C for 5 minutes; ii) 98°C for 20 seconds; iii) 58°C for 20 seconds; and iv) 72°C for 3 minutes. Steps ii) to iv) were repeated for 15 cycles, followed by 72°C for 5 minutes. The resulting mixture was removed and placed on ice. The amplified cDNA concentration was assayed using Qubit dsDNA Mix containing 198 µL of Invitrogen^{™} Qubit dsDNA HS Buffer, 1 µL of Qubit dsDNA HS Reagent (200×), and 1 µL of cDNA product.
(3) cDNA purification: The PCR product from the previous step was transferred to a new 1.5 mL centrifuge tube, and mixed with VAHTS^{™} DNA Clean Beads (VAZYME) equilibrated at room temperature at a volume ratio of 1:1. The mixture was shaken and mixed well, and incubated at room temperature for 10 minutes. After a brief centrifugation, the EP tube was placed on a magnetic rack for 3 minutes until the solution was clarified, and the supernatant was discarded. 1 mL of freshly prepared 80% ethanol was added, and the mixture was allowed to stand for 30 seconds. The supernatant was discarded, and the tube was washed again with 80% ethanol. The supernatant was discarded, and the mixture was air-dried at room temperature until the surface of the magnetic beads was free of reflection and showed no cracks. 40 µL of NF-H₂O was added for re-dissolution. The mixture was shaken and mixed well, and allowed to stand at room temperature for 5 minutes. After a brief centrifugation, the tube was placed on a magnetic rack for 3 to 5 minutes until the solution was clarified, and the supernatant was transferred to a new 1.5 mL centrifuge tube. 1 µL of cDNA sample was tested for concentration using Qubit dsDNA HS Kit, and cDNA fragment distribution was analyzed using Agilent 2100 High Sensitivity DNA Kit. (QC criteria: Fragment size is mainly distributed in 1000 to 1500 bp)

### 1.9 cDNA library circularization, sequencing, and data analysis

50 ng of the purified product was added to a new PCR tube, supplemented with NF-H₂O to a total volume to 20 µL, and added with 20 µL of 2× Make DNB buffer. The mixture was reacted in a PCR instrument: 95°C for 3 minutes, and 40°C for 3 minutes. The reaction mixture was removed and placed on ice, followed by the addition of 39 µL of RCA buffer, 1 µL of 10 mM ATP, and 4 µL of One Step Enzyme. The mixture was reacted in the PCR instrument at 30°C for 30 minutes. The reaction mixture was removed and added with 20 µL of DNB stop buffer to obtain DNB libraries. The libraries were sequenced on a MGISEQ-2000RS sequencer. The sequencing data was automatically analyzed on the web page (*https:*//*luat.stomics.tech*/*sap*/) to generate heat map results.

### Example 2

Steps 2.1 to 2.4 were identical to steps 1.1 to 1.4 of Example 1.

### 2.5 Hybridization of random probe (6N) with splint

A primer hybridization mixture was prepared during the fixation of tissue sections.

10 µM random probe 6N (5'pho-SEQ ID NO: 4-3') and 10 µM splint (5'-/rU/SEQ ID NO: 5-3') were mixed in a PCR tube, followed by gradient cooling using a PCR instrument from 95°C to 25°C at a rate of 2°C per minute, and then removed and placed on ice. 1 µL of the primer hybridization mixture was added to 5× SSC, followed by the addition of 5% by volume of RNase inhibitor, mixed well, and placed on ice.

After the permeabilization reaction was completed, the chip was removed, and the liquid was aspirated from the chip surface. The chip was washed once with a mixture solution of 190 µL 5× SSC and 10 µL RI, added with the prepared primer hybridization mixture, and hybridized at room temperature for 15 minutes. The chip was then washed once with 5× SSC and RI. A primer hybridization mixture was obtained, as shown in Group 4 of FIG. 3.

Steps 2.6 to 2.9 were identical to steps 1.6 to 1.9 of Example 1.

### Comparative Example 1

Steps 1.1 to 1.4 were identical to steps 1.1 to 1.4 of Example 1.

### 1.5 Hybridization of random probe (6N) with splint

A primer hybridization mixture was prepared during the fixation of tissue sections. 5 µM random probe 6N (5'pho-SEQ ID NO: 2-3') and 5 µM splint (5'-SEQ ID NO: 3-3') were added to 5× SSC, mixed well, and hybridized in an incubator at 55°C for 10 minutes. After removal, the mixture was placed on ice, added with 5% by volume of RNase inhibitor (RI), mixed well, and placed on ice.

After the permeabilization reaction was completed, the chip was removed, and the liquid was aspirated from the chip surface. The chip was washed once with a mixture solution of 190 µL 5× SSC and 10 µL RI, added with the prepared primer hybridization mixture, and hybridized at room temperature for 15 minutes. The chip was then washed once with 5× SSC and RI. A primer hybridization mixture was obtained, as shown in Group 1 of FIG. 3.

Steps 1.6 to 1.9 were identical to steps 1.6 to 1.9 of Example 1.

### Comparative Example 2

Steps 2.1 to 2.4 were identical to steps 1.1 to 1.4 of Example 1.

### 2.5 Hybridization of random probe (6N) with splint

A primer hybridization mixture was prepared during the fixation of tissue sections.

10 µM random probe 6N (5'pho-SEQ ID NO: 2-3') and 10 µM splint (5'-SEQ ID NO: 3-3') were mixed in a PCR tube, followed by gradient cooling using a PCR instrument from 95°C to 25°C at a rate of 2°C per minute, and then removed and placed on ice. 1 µL of the primer hybridization mixture was added to 5× SSC, followed by the addition of 5% by volume of RNase inhibitor, mixed well, and placed on ice.

After the permeabilization reaction was completed, the chip was removed, and the liquid was aspirated from the chip surface. The chip was washed once with a mixture solution of 190 µL 5× SSC and 10 µL RI, added with the prepared primer hybridization mixture, and hybridized at room temperature for 15 minutes. The chip was then washed once with 5× SSC and RI. A primer hybridization mixture was obtained, as shown in Group 2 of FIG. 3.

Steps 2.6 to 2.9 were identical to steps 1.6 to 1.9 of Example 1.

### Conclusion

As shown in FIG. 3, spatial transcriptomic capture tests were performed on both OCT-embedded fresh mouse brain hemisphere sections (A) and paraffin-embedded mouse brain hemisphere sections (B), and the number of adapters generated by the splint was analyzed for each method.

Valid reads: Nucleic acid sequences that, after sequencing and algorithmic analysis, are determined to have been generated by random probe capture, reverse transcription and ligation to the chip.

Adapter generated by splint: As described in the principle, a sequence called an adapter sequence that is generated due to the strand displacement function of RTase .

Unique mapping: Sequences in the valid reads that can be mapped to a unique position in the genome after being mapped to the reference genome.

Panel A shows the test data of OCT-embedded fresh mouse brain. Data analysis revealed that with approximately consistent valid reads (both around 210M), the proportion of splint-induced adapters decreased from 45% to 20% using 5'pho-SEQ ID NO: 4-3' and 5'-/rU/SEQ ID NO: 5-3', showing a decrease of approximately 25%. Consequently, the proportion of Unique mapping increased from approximately 2.5% to approximately 8.5%, showing an increase of 3.4 times. The results demonstrated that 5'pho-SEQ ID NO: 4-3' and 5'-/rU/SEQ ID NO: 5-3' reduced the number of splint-induced adapters and enhanced the proportion of Unique mapping, regardless of the hybridization method.

Panel B shows the test data of paraffin-embedded (FFPE) mouse brain. Data analysis revealed that with approximately consistent valid reads, the proportion of splint-induced adapters decreased from 50% to 22% using 5'pho-SEQ ID NO: 4-3' and 5'-/rU/SEQ ID NO: 5-3', showing a decrease of approximately 28%. Consequently, the proportion of Unique mapping increased from approximately 1% to approximately 2%, showing an increase of 2 times. The conclusion was the same as that of the OCT-embedded fresh mouse brain.

Although the specific embodiments of the present disclosure have been described above, those skilled in the art should understand that these are merely illustrative examples, and various changes or modifications may be made to these embodiments without departing from the principles and essence of the present disclosure. Therefore, the scope of protection of the present disclosure is defined by the appended claims.

## Claims

1. A splint sequence, wherein the first base at the 5' end of the splint sequence is U; preferably, the nucleotide sequence following the first base at the 5' end of the splint sequence is as shown in SEQ ID NO: 5.

2. A nucleic acid capture kit, comprising the splint sequence according to claim 1; preferably, the nucleic acid capture kit further comprises at least one selected from the group consisting of a random probe, a T4 ligase, a reverse transcriptase, a buffer for ligation, or a buffer for reverse transcription.

3. Use of the splint sequence according to claim 1 in nucleic acid capture.

4. A method for capturing nucleic acid, comprising reverse transcribing an RNA in a sample captured by a random probe into a cDNA, and ligating the cDNA to the random probe by means of hybridization of the splint sequence according to claim 1 to the random probe and a fixed oligonucleotide sequence, to form a long single-stranded nucleotide sequence containing the oligonucleotide sequence, the random probe, and the cDNA in the 5' to 3' direction, wherein the reverse transcription and the ligation are performed in the same reaction system.

5. The method according to claim 4, wherein the method comprises the following steps:
(1) hybridizing the random probe to the splint sequence; wherein the 5' end of the random probe is partially complementary to the 5' end of the splint sequence;
(2) hybridizing the random probe to the RNA in the sample; wherein the 3' end of the random probe is partially complementary to the 3' end of the RNA;
(3) hybridizing the splint sequence to the fixed oligonucleotide sequence; preferably, the oligonucleotide sequence is fixed on a capture chip; and
(4) in the same reaction system, synthesizing the cDNA using the RNA in the complex formed by simultaneous hybridization in steps (1), (2), and (3) as a template, and ligating the fixed oligonucleotide sequence to the random probe in the complex, allowing the fixed oligonucleotide sequence, the random probe, and the cDNA to form a long single-stranded nucleotide sequence containing cDNA in the 5' to 3' direction.

6. The method according to claim 5, wherein the capture chip is reacted at 30 to 45°C, such as 37°C, for 3 to 24 hours, such as 3 to 5 hours;
and preferably, in step (1), the random probe comprises 6 to 20 N.

7. The method according to claim 4 or 5, wherein the method further comprises:
(i) mixing a random probe and the splint sequence with 5× SSC, performing a hybridization in an incubator at 50 to 60°C, such as 55°C, for 5 to 30 minutes, such as 10 minutes, to form a primer hybridization mixture, removing and cooling the primer hybridization mixture, and adding 2 to 15% by volume, such as 5% by volume of RNase inhibitor; or
(i') mixing the random probe and the splint sequence in a solution, gradient cooling to room temperature, followed by cooling to 0 to 4°C to obtain a primer hybridization mixture, and mixing the primer hybridization mixture with 5× SSC and 2 to 15% by volume, such as 5% by volume of RNase inhibitor.

8. The method according to claim 7, wherein after step (i) or step (i'), the method further comprises:
(ii) aspirating the liquid from the surface of a capture chip, washing the capture chip with a mixture 2, adding the primer hybridization mixture to the chip surface, performing a hybridization at room temperature for 15 to 60 minutes, and washing the capture chip again with a mixture 2; the mixture 2 contains 5× SSC and RNase inhibitor.

9. The method according to claim 8, wherein
in step (i'), the gradient cooling is performed from 90 to 100°C to 20 to 28°C at a rate of 1 to 5°C per minute;
in step (i) or (i'), the random probe is 6N;
in step (i) or (i'), the molar ratio of the random probe to the splint sequence is (1 to 10):(1 to 10); and/or,
in step (ii), the volume ratio of 5× SSC to RNase inhibitor in the mixture 2 is (5 to 30):1.

10. The method according to claim 9, wherein
in step (i'), the gradient cooling is performed from 95°C to 25°C at a rate of 2°C per minute;
in step (i) or (i'), the molar ratio of the random probe to the splint sequence is 1:1; and/or,
in step (ii), the volume ratio of 5× SSC to RNase inhibitor in the mixture 2 is 19:1.

11. The method according to any one of claims 4 to 10, wherein the capture chip comprises a capture probe, wherein the capture probe comprises spatial barcode location information and a fixed oligonucleotide sequence; the fixed oligonucleotide sequence is hybridized to the 3' end of the splint sequence.

12. The method according to claim 11, wherein the capture probe is 5'Nm-SEQ ID NO: 1-3', and the m is 8 to 30.

13. The method according to claim 12, wherein the m is 25 or more.

14. A high-throughput sequencing method, comprising the following steps:
(I) obtaining a long single-stranded nucleotide sequence containing cDNA to be sequenced according to the method according to any one of claims 3 to 13;
(II) constructing a cDNA library;
(III) sequencing the library and analyzing the obtained data.

15. The method according to claim 14, wherein step (I) is followed by the steps of cDNA fragmentation, amplification, and purification; and/or, in step (II), the cDNA library is circularized to obtain a circularized library of DNB.
